# EUROPEAN PATENT APPLICATION

(11) **EP 4 817 669 A1**
(43) Date of publication of application: **07.10.2026**
(21) Application number: 24910480.3
(22) Date of filing: 27.11.2024
(51) Int. Cl.: A61M 60/861, A61M 60/863, A61M 60/865

(54) **VENTRICULAR CONNECTION MECHANISM AND VENTRICULAR ASSISTANCE SYSTEM**

(30) Priority: 30.12.2023 CN 202311868794
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YAO, Yixin, Shenzhen, Guangdong 518000 (CN); YU, Shunzhou, Shenzhen, Guangdong 518000 (CN)
(74) Representative: Appleyard Lees IP LLP
(86) International application number: PCT/CN2024/134781
(87) International publication number: WO 2025/139547

(57) **Abstract**

A ventricular connection mechanism (1) and a ventricular assistance system. The ventricular connection mechanism (1) comprises a suture ring (10), a clamping assembly (20), and a stopper (50). The clamping assembly (20) comprises an annular disc (30) and a clamping ring (22). The annular disc (30) is provided with a first opening (363). The clamping ring (22) is fixedly connected to the annular disc (30) and provided with a second opening (21) corresponding to the position of the first opening (363). The size of the second opening (21) is adjustable. The stopper (50) is connected to the annular disc (30), and the position of the stopper corresponds to the position of the first opening (363), so that the stopper (50) and the annular disc (30) together form a closed annular structure (100). A fixing structure (200) of the annular structure (100) can allow the annular structure (100) to be fixedly connected to the suture ring (10), and the fixing structure is disposed around an inner hole of the suture ring (10).

## Description

### CROSS-REFERENCE TO RELATED APPLICATIONS

This application claims priority of Chinese Patent Application No. 202311868794.9, filed on December 30, 2023, the content of which is hereby incorporated by reference in its entirety.

### TECHNICAL FIELD

The present application relates to the technical field of medical devices, and in particular to a ventricular connection mechanism and a ventricular assist system.

### BACKGROUND

Blood pumps can assist the heart in pumping blood and are commonly used to assist in the treatment of heart failure. During the implantation surgery of blood pumps, a ventricular connection mechanism is usually required to fix the blood pump to heart tissue. However, conventional ventricular connection mechanisms are prone to blood leakage after the blood pump is mounted, which needs to be improved.

### SUMMARY

Based on this, the present application provides a ventricular connection mechanism and a ventricular assist system that can reduce the probability of blood leaking from a first opening of an annular disk and a gap between a suture ring and the annular disk.

In a first aspect, a ventricular connection mechanism is provided according to an embodiment of the present application, and the ventricular connection mechanism includes:
a suture ring;
a clamping assembly including an annular disk and a clamping ring fixedly connected to the annular disk, the annular disk having a first opening, the clamping ring having a second opening, a size of the second opening being adjustable, and an inner diameter of the clamping ring being adjustable by adjusting the size of the second opening; and
a blocking member connected to the annular disk, a position of the blocking member corresponding to a position of the first opening, such that the blocking member and the annular disk cooperatively form a closed annular structure, the annular structure being provided with a fixing structure capable of fixedly connecting the annular structure to the suture ring, and the fixing structure being provided around an inner hole of the suture ring.

In a second aspect, a ventricular assist system is provided according to an embodiment of the present application. The ventricular assist system includes a ventricular assist device and the ventricular connection mechanism described above, the ventricular assist device includes an inlet pipe capable of extending through the clamping ring, and the clamping ring is capable of clamping the inlet pipe.

Details of one or more embodiments of the present application are set forth in the following drawings and descriptions. Other features, objects and advantages of the present application will become apparent with reference to the specification, drawings, and claims.

### BRIEF DESCRIPTION OF THE DRAWINGS

In order to illustrate the embodiments of the present application more clearly, the drawings used in the embodiments will be described briefly. Apparently, the following described drawings are merely for the embodiments of the present application, and other drawings can be derived by those of ordinary skill in the art without any creative effort.
FIG. 1 is a schematic structural view of a ventricular connection mechanism provided in one embodiment.
FIG. 2 is a schematic structural view of the ventricular connection mechanism shown in FIG. 1 with a suture ring omitted.
FIG. 3 is a rear view of the ventricular connection mechanism shown in FIG. 2.
FIG. 4 is a partial exploded view of the ventricular connection mechanism shown in FIG. 3.
FIG. 5 is a schematic structural view of a blocking member of the ventricular connection mechanism shown in FIG. 1 from one perspective.
FIG. 6 is a schematic structural view of the blocking member shown in FIG. 5 from another perspective.
FIG. 7 is a front view of the ventricular connection mechanism shown in FIG. 2.
FIG. 8 is a partial enlarged view of part A of the ventricular connection mechanism shown in FIG. 7.
FIG. 9 is a cross-sectional view taken along the line X-X of the ventricular connection mechanism shown in FIG. 7.
FIG. 10 is a partial enlarged view of part B of the ventricular connection mechanism shown in FIG. 9.

### DETAILED DESCRIPTION OF THE EMBODIMENTS

The present application will now be described in detail with reference to the accompanying drawings and embodiments in order to make the objects, technical solutions, and advantages of the present application clearer. It should be understood that the specific embodiments described herein are only for explaining the present application, and not intended to limit the present application.

It should be noted that when an element is called "fixed to" or "provided on" another element, it can be directly on another element or there can be a centered element. When an element is considered to be "connected" to another element, it can be directly connected to another element or indirectly connected to the other element.

In addition, the terms "first" and "second" are only used for descriptive purposes, and should not be construed as indicating or implying relative importance or implying the number of indicated technical features. Thus, a feature delimited with "first", "second" may expressly or implicitly include at least one of the features. In the description of the present application, "plurality" means two or more, unless otherwise expressly and specifically defined.

In order to illustrate the technical solution of the present application, the specific drawings and embodiments will be combined to illustrate below.

In blood pump implantation surgery, a ventricular connection mechanism is usually used to fix a blood pump to a heart. Specifically, the ventricular connection mechanism is fixed to an outer wall of the heart, an inlet pipe of the blood pump extends through the ventricular connection mechanism and extends into a ventricle, and the inlet pipe is locked by the ventricular connection mechanism. The inventors of the present application have discovered that, due to the unevenness of the heart surface and the fact that doctors pull on the ventricular connection mechanism during implantation, and the lack of structural support at the position of the suture ring corresponding to an opening of the annular disk, the suture ring is prone to deformation at the opening of the annular disk, and a gap between the annular disk and the suture ring of the ventricular connection mechanism increases, causing the blood pump to be prone to blood leakage after being mounted in a conventional ventricular connection mechanism.

In order to improve at least some of the aforementioned problems, the present application provides a ventricular connection mechanism and a ventricular assist system. By connecting a blocking member to an annular disk, and the position of the blocking member corresponding to the position of the opening of the annular disk, the blocking member and the annular disk cooperatively form a closed annular structure. The annular structure is provided with a fixing structure capable of fixedly connecting the annular structure to a suture ring. The fixing structure surrounds an inner hole of the suture ring, so that the annular structure can support the suture ring around the inner hole of the suture ring, which enhances the structural stability of the suture ring. Thus, during the process of suturing the suture ring to the heart, the problem of increased gap between the suture ring and the annular disk caused by deformation of the suture ring can be reduced, and the probability of blood leaking from the opening of the annular disk and the gap between the suture ring and the annular disk can be reduced. The ventricular connection mechanism and ventricular assist system provided in the present application will be described in detail below with reference to specific embodiments and accompanying drawings.

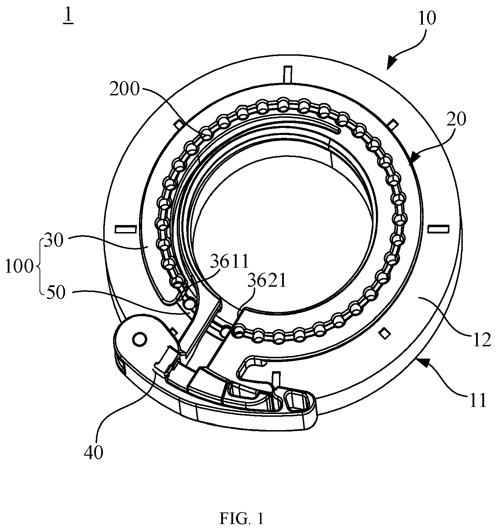

Referring to FIGS. 1 to 3, an embodiment of the present application provides a ventricular connection mechanism 1 configured to fix a ventricular assist device on a heart. Specifically, the ventricular connection mechanism 1 according to one embodiment includes a suture ring 10, a clamping assembly 20, and a blocking member 50.

The suture ring 10 is configured to be fixed on the heart, and in some embodiments, the suture ring 10 is sutured to the heart through a suture thread. Furthermore, the suture ring 10 is of an annular structure with an inner hole, and the inner hole of the suture ring 10 is configured to allow an inlet pipe of the ventricular assist device to extend through.

The suture ring 10 is substantially of a flat annular structure. The suture ring 10 includes a first suturing surface 11 and a second suturing surface 12 opposite to each other. The first suturing surface 11 is capable of abutting against an outer wall of the heart. The second suturing surface 12 is provided on a side of the suture ring 10 away from the first suturing surface 11.

In this embodiment, the suture ring 10 is made of a flexible material, which is beneficial for the suture ring 10 to better conform to the outer wall of the heart. Specifically, the suture ring 10 may include a silicone member and a suturing cloth covering the silicone member, that is, the first suturing surface 11 and the second suturing surface 12 may be two surfaces of the suturing cloth covering the silicone member in an axial direction of the suture ring 10. The axial direction of the suture ring 10 refers to an extension direction of a central axis of the suture ring 10. The silicone member has good biocompatibility and is easy for a suture thread to extend through, thereby facilitating the suture ring 10 to be sutured to the heart. In other embodiments, the silicone member in the suture ring 10 can also be replaced with TPE (Thermoplastic elastomer), soft PVC (Polyvinyl chloride), etc.

Continuing to refer to FIGS. 1, 3, and 4, the clamping assembly 20 includes an annular disk 30 and a clamping ring 22. The annular disk 30 is fixedly connected to the second suturing surface 12 of the suture ring 10. The annular disk 30 has a first opening 363. The annular disk 30 further has a first end 361 and a second end 362 that form the first opening 363. The first end 361 and the second end 362 are opposite to each other along a circumferential direction of the annular disk 30, and the first end 361 and the second end 362 are spaced apart to form the first opening 363. The circumferential direction of the annular disk 30 refers to a circular direction of the annular disk 30. The annular disk 30 is of a rigid structure, which can be made of stainless steel, titanium alloy, etc.

The clamping ring 22 is fixedly connected to the annular disk 30. The clamping ring 22 and the annular disk 30 are approximately coaxial. Specifically, the clamping ring 22 is provided on an inner periphery of the annular disk 30.

The clamping ring 22 has a second opening 21, and a size of the second opening 21 is adjustable. The inner diameter of the clamping ring 22 can be adjusted by adjusting the size of the second opening 21, so that the clamping ring 22 has a certain elastic deformation ability, that is, the clamping ring 22 can contract or expand radially to adjust its own inner diameter. The inlet pipe of the ventricular assist device (not shown) can extend through the clamping ring 22. The clamping ring 22 is a ring with an opening, and the second opening 21 is an opening in the circumferential direction of the clamping ring 22. The circumferential direction of the clamping ring 22 refers to the circular direction of the clamping ring 22, and the radial direction of the clamping ring 22 refers to a direction along a diameter or radius of the clamping ring 22. In the illustrated embodiment, the second opening 21 may be a part of the first opening 363. In other embodiments, an end of the clamping ring 22 in the axial direction of the suture ring 10 is fixedly connected to the annular disk 30.

The clamping assembly 20 further includes a first connecting arm 23 and a second connecting arm 24. The first connecting arm 23 is connected to one end of the clamping ring 22, and the second connecting arm 24 is connected to the other end of the clamping ring 22. The first connecting arm 23 can approach or move away from the second connecting arm 24 to adjust the size of the second opening 21, thereby adjusting the inner diameter of the clamping ring 22.

In some embodiments, both the first connecting arm 23 and the second connecting arm 24 are connected to an outer side of the clamping ring 22. In some embodiments, the first connecting arm 23 and the second connecting arm 24 are connected to two ends of the clamping ring 22 in the circumferential direction, respectively. In this case, the second opening 21 is provided between the first connecting arm 23 and the second connecting arm 24. In some embodiments, the second connecting arm 24 is also connected to the second end 362 of the annular disk 30 and extends outward from the first opening 363.

The blocking member 50 is fixedly provided on the second suturing surface 12 of the suture ring 10. For example, the blocking member 50 is sutured to the suture ring 10 by a suture thread. The blocking member 50 is connected to the annular disk 30, and the position of the blocking member 50 corresponds to the position of the first opening 363, such that the blocking member 50 and the annular disk 30 cooperatively form a closed annular structure 100. The position of the blocking member 50 corresponds to the position of the first opening 363, which means that the blocking member 50 is provided at the first opening 363 of the annular disk 30.

The annular structure 100 is provided with a fixing structure 200, which can fix the annular structure 100 to the suture ring 10. The fixing structure 200 is provided around the inner hole of the suture ring 10.

In this embodiment, the annular disk 30 is provided with a plurality of first suturing holes 33. The blocking member 50 is provided with a plurality of second suturing holes 53. The ventricular connection mechanism 1 further includes a suture thread (not shown). The suture thread extends through the plurality of first suturing holes 33, the plurality of second suturing holes 53, and the suture ring 10 to fix the annular structure 100 to the suture ring 10. That is, both the annular disk 30 and the blocking member 50 can both be connected to the suture ring 10 by the suture thread. The plurality of first suturing holes 33, the plurality of second suturing holes 53, and the suture thread cooperatively constitute the fixing structure 200.

In other embodiments, the fixing structure 200 may also be an adhesive provided between the annular structure 100 and the suture ring 10, that is, the annular structure 100 is bonded to the suture ring 10 through an adhesive. In some embodiments, the fixing structure 200 may also be other structures capable of fixing the annular structure 100 to the suture ring 10.

In this embodiment, along the axial direction of the suture ring 10, the blocking member 50 is located between the suture ring 10 and the first connecting arm 23, so that the first connecting arm 23 can extend outward from a side of the blocking member 50 away from the suture ring 10 through the first opening 363. The blocking member 50 does not block the movement of the first connecting arm 23, so that the first connecting arm 23 can move along the circumferential direction of the annular disk 30 within the first opening 363 to adjust the inner diameter of the clamping ring 22, thereby clamping the inlet pipe of the ventricular assist device.

The blocking member 50 includes a first connecting end 541 and a second connecting end 551 opposite to each other. The first connecting end 541 is connected to the first end 361 of the annular disk 30, and the second connecting end 551 is connected to the second end 362 of the annular disk 30, so that the blocking member 50 can fill the first opening 363 of the annular disk 30, and the annular disk 30 and the blocking member 50 cooperatively form the closed annular structure 100.

In the aforementioned ventricular connection mechanism 1, the inner diameter of the clamping ring 22 is adjusted by the size of the second opening 21, so that the clamping ring 22 can clamp the inlet pipe of the ventricular assist device. The blocking member 50 is connected to the annular disk 30, and the position of the blocking member 50 corresponds to the position of the first opening 363, so that the blocking member 50 and the annular disk 30 cooperatively form the closed annular structure 100. The annular structure 100 is provided with the fixing structure 200 that can fix the annular structure 100 to the suture ring 10, and the fixing structure 200 is provided around the inner hole of the suture ring 10, so that the annular structure 100 can support the suture ring 10 around the inner hole of the suture ring 10, thereby enhancing the structural stability of the suture ring 10. Thus, during the process of suturing the suture ring 10 to the heart, the problem of increased gap between the suture ring 10 and the annular disk 30 due to deformation of the suture ring 10 can be addressed, and the probability of blood leaking from the first opening 363 of the annular disk 30 and the gap between the suture ring 10 and the annular disk 30 can be reduced.

Continuing to refer to FIGS. 1, 3, and 4, the annular disk 30 surrounds and is connected to the clamping ring 22. Specifically, the annular disk 30 has a first semi-ring 31 and a second semi-ring 32 connected to each other. A junction of the first semi-ring 31 and the second semi-ring 32 is provided opposite to the first opening 363 along the radial direction of the annular disk 30. The first end 361 is located at an end of the first semi-ring 31 away from the second semi-ring 32, and the second end 362 is located at an end of the second semi-ring 32 away from the first semi-ring 31.

Furthermore, the first semi-ring 31 is spaced apart from the clamping ring 22, and the second semi-ring 32 is fixedly connected to the clamping ring 22. In this way, the elastic deformation ability of the clamping ring 22 is ensured, and the clamping ring 22 is prevented from being unable to contract after the blocking member 50 is connected to the first end 361 and the second end 362 of the annular disk 30.

The annular disk 30 includes a disk body 311 and a boss 321 provided on the disk body 311. A part of the boss 321 and the disk body 311 form the first semi-ring 31, and another part of the boss 321 and the disk body 311 form the second semi-ring 32.

The boss 321 is provided on the inner ring of the disk body 311. The inner peripheral surface of the boss 321 and the inner peripheral surface of the disk body 311 form the same circumferential surface. The boss 321 can be in contact with the suture ring 10. The first suturing hole 33 is provided on the boss 321. For example, the boss 321 is connected to the second suturing surface 12 of the suture ring 10. The disk body 311 is spaced apart from the suture ring 10 to facilitate gripping the ventricular connection mechanism 1 by a clamp. The disk body 311 can be in contact with a housing of the ventricular assist device.

Referring to FIGS. 1 to 3, in an embodiment, the ventricular connection mechanism 1 further includes a locking assembly 40. The locking assembly 40 is connected to the clamping assembly 20. In the illustrated embodiment, the locking assembly 40 can rotatably abut against the first connecting arm 23. When the locking assembly 40 rotates, it can cause the first connecting arm 23 to move toward the second connecting arm 24, thereby reducing the size of the second opening 21 and reducing the inner diameter of the clamping ring 22. The locking assembly 40 is also engaged with the second connecting arm 24 to maintain the inner diameter of the clamping ring 22. In an embodiment, the locking assembly 40 may also be connected to both the first connecting arm 23 and the second connecting arm 24. For example, the locking assembly 40 is rotatably provided on the first connecting arm 23 and is engaged with the second connecting arm 24.

The locking assembly 40 has a locked state and a released state. When the locking assembly 40 is engaged with the second connecting arm 24, the locking assembly 40 is in the locked state, the locking assembly 40 can drive the first connecting arm 23 to move toward the second connecting arm 24, so that the clamping ring 22 of the clamping assembly 20 contracts radially, thereby enabling the clamping ring 22 to clamp the inlet pipe extending therethrough. When the engagement between the locking assembly 40 and the second connecting arm 24 is released, the locking assembly 40 is in the unlocked state, the contraction state of the clamping ring 22 can be released, and the clamping ring 22 expands radially under its own elastic force to release the inlet pipe.

When implanting the ventricular assist device, the suture ring 10 is first sutured to the heart. Then, the inlet pipe of the ventricular assist device extends through the suture ring 10 and the clamping ring 22. Finally, the locking assembly 40 drives the first connecting arm 23 to move toward the second connecting arm 24, so that the clamping ring 22 contracts radially, enabling the clamping ring 22 to clamp the inlet pipe of the ventricular assist device and thus fixing the ventricular assist device to the heart.

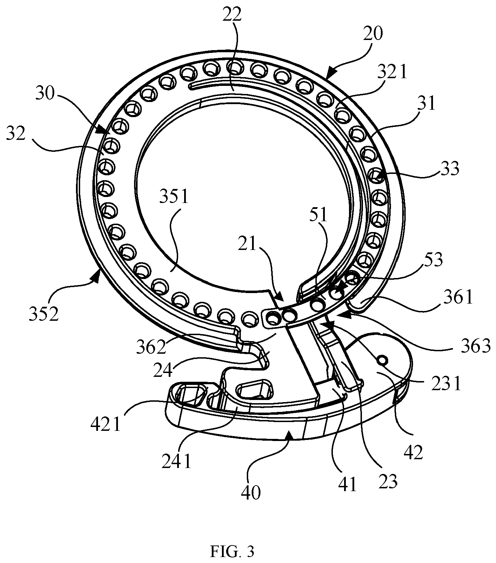

Referring to FIGS. 1 and 3, in an embodiment, the locking assembly 40 includes a base body 41 and a locking member 42. The base body 41 is connected to the second connecting arm 24. One end of the locking member 42 is rotatably connected to the base body 41, and the other end of the locking member 42 is provided with an engaging portion 421 configured to be engaged with the second connecting arm 24. Specifically, the second connecting arm 24 is provided with a curved surface 241, and the engaging portion 421 is configured to be engaged with the curved surface 241.

When the locking member 42 is driven to rotate relative to the base body 41, the end of the locking member 42 adjacent to the base body 41 pushes the first connecting arm 23 to move toward the second connecting arm 24, thereby causing the clamping ring 22 to contract radially inward. That is, the end of the locking member 42 adjacent to the base body 41 rotatably abuts against the first connecting arm 23, and when the locking member 42 rotates relative to the base body 41, the first connecting arm 23 can be pushed to move toward the second connecting arm 24 to reduce the second opening 21 and reduce the inner diameter of the clamping ring 22. The locking member 42 is driven to continue to rotate, and when the clamping ring 22 contracts to clamp the inlet pipe, and the engaging portion 421 is in contact with the curved surface 241 of the second connecting arm 24, the engagement between the engaging portion 421 and the second connecting arm 24 can maintain the clamping ring 22 in the state of clamping the inlet pipe. When it is necessary to release the inlet pipe, by releasing the engagement between the engaging portion 421 and the second connecting arm 24, the locking assembly 40 is set to the released state, thereby releasing the contraction state of the clamping ring 22, allowing the clamping ring 22 to expand radially under its own elastic force to release the inlet pipe. In other embodiments, the locking assembly 40 is rotatably abutted against the first connecting arm 23 and is connected to the second connecting arm 24, and the locking assembly 40 can drive the first connecting arm 23 and the second connecting arm 24 to approach each other, so that the clamping ring 22 contracts radially inward, and then the clamping ring 22 clamps the inlet pipe of the ventricular assist device. It should be understood that there are many other ways for the locking assembly 40 to drive the first connecting arm 23 and the second connecting arm 24 to approach or move away from each other, which are not limited to the above embodiments and will not be described in detail.

In this embodiment, the blocking member 50 is fixedly connected to both the suture ring 10 and the annular disk 30. In the illustrated embodiment, the blocking member 50 is welded and fixed to the annular disk 30. It should be understood that in other embodiments, the blocking member 50 may also be connected to the annular disk 30 by an adhesive or the like. In this embodiment, both the blocking member 50 and the annular disk 30 are rigid structures, which is beneficial to supporting the suture ring 10 and reducing deformation of the suture ring 10 during suturing. For example, both the blocking member 50 and the annular disk 30 may be made of titanium alloy or stainless steel. Preferably, the blocking member 50 and the annular disk 30 can be made of the same material, which makes it easier to weld the blocking member 50 to the annular disk 30 and avoids the problem of difficulty in welding between different materials.

Referring to FIGS. 3, 5, and 6, optionally, in an embodiment, both the blocking member 50 and the annular disk 30 are arc-shaped, and a central axis of a circle corresponding to an arc where the blocking member 50 is located is coaxial with a central axis of a circle where the annular disk 30 is located. In this way, the shape of the blocking member 50 can better fit the shape of the annular disk 30, and the blocking member 50 and the annular disk 30 can enclose a complete ring. When the first connecting end 541 of the blocking member 50 and the second connecting end 551 are respectively connected to the first end 361 and the second end 362 of the annular disk 30, that is, when the first connecting end 541 of the blocking member 50 is connected to the first end 361 of the annular disk 30 and the second connecting end 551 of the blocking member 50 is connected to the second end 362 of the annular disk 30, when viewed from a top view of the ventricular connection mechanism 1, the blocking member 50 and the annular disk 30 can combine to form a complete annular structure, which makes the ventricular connection mechanism 1 more concise as a whole.

Referring to FIGS. 1, 4, and 5, optionally, in an embodiment, the first end 361 and the second end 362 of the annular disk 30 are each provided with the first suturing hole 33. Specifically, the first suturing hole 33 is configured to allow the suture thread to extend through, so as to facilitate the suturing connection between the annular disk 30 and the suture ring 10. The first connecting end 541 and the second connecting end 551 of the blocking member 50 are each provided with the second suturing hole 53.

For ease of description, the first suturing hole closest to the first end 361 and the first suturing hole closest to the second end 362 are defined as first suturing holes 33a, and other first suturing holes are defined as first suturing holes 33b. The second suturing hole closest to the first connecting end 541 and the second suturing hole closest to the second connecting end 551 are defined as second suturing holes 53a, and the second suturing holes (i.e., other second suturing holes) located at the first opening 363 are defined as second suturing holes 53b.

Furthermore, the first suturing hole 33a at the first end 361 is coaxial with the second suturing hole 53a at the first connecting end 541, and the first suturing hole 33a at the second end 362 is coaxial with the second suturing hole 53a at the second connecting end 551. It avoids that the first suturing holes 33a located at the first end 361 and the second end 362 are blocked by the blocking member 50, and avoids that the second suturing holes 53a located at the first connecting end 541 and the second connecting end 551 are blocked by the annular disk 30, thereby facilitating the suturing connection of the annular disk 30 and the blocking member 50 to the suture ring 10.

In this embodiment, when the first connecting arm 23 approaches the second connecting arm 24, that is, when the locking assembly 40 is in the locked state, at least one second suturing hole 53b can be exposed from the first opening 363, thus preventing the first connecting arm 23 from blocking the at least one second suturing hole 53b. In order to facilitate suturing connection of the blocking member 50 and the annular disk 30 to the suture ring 10, the suturing operation is usually performed when the locking assembly 40 is in the locked state. When the locking assembly 40 is in the locked state, at least one second suturing hole 53b can be exposed from the first opening 363, which is beneficial for the suturing operation of the blocking member 50 and the suture ring 10 at the first opening 363. It should be noted that during the movement of the first connecting arm 23 approaching the second connecting arm 24, it does not block other second suturing holes 53.

In an embodiment, the first suturing hole 33a at the first end 361 is coaxial with the second suturing hole 53a at the first connecting end 541, and the first suturing hole 33a at the second end 362 is not coaxial with the second suturing hole 53a at the second connecting end 551. In another embodiment, the first suturing hole 33a at the first end 361 is not coaxial with the second suturing hole 53a at the first connecting end 541, and the first suturing hole 33a at the second end 362 is coaxial with the second suturing hole 53a at the second connecting end 551.

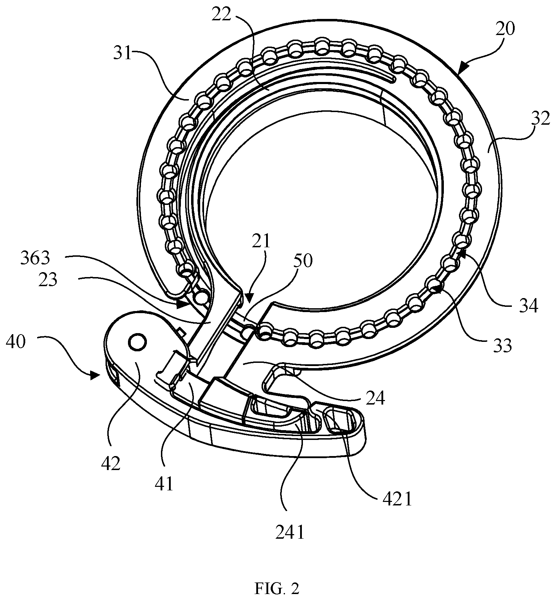

Referring to FIGS. 2 and 3, furthermore, the annular disk 30 is further provided with a plurality of first recessed grooves 34 capable of accommodating the suture thread. One first suturing hole 33 is provided between adjacent two first recessed grooves 34. The first recessed groove 34 is in communication with adjacent two first suturing holes 33, that is, one first recessed groove 34 is provided between adjacent two first suturing holes 33. In this way, the suture thread extending from the first suturing hole 33 can extend into the next first suturing hole 33 along the first recessed groove 34, thereby avoiding the suture thread from protruding from the surface of the annular disk 30 and affecting the mounting of the ventricular assist device.

Referring to FIGS. 1, 6, and 7, in an embodiment, the blocking member 50 is further provided with a plurality of second recessed grooves 58 capable of accommodating the suture thread. One second suturing hole 53 is provided between adjacent two second recessed grooves 58. The second recessed groove 58 is in communication with adjacent two second suturing holes 53, that is, one second recessed groove 58 is provided between adjacent two second suturing holes 53. In this way, the suture thread extending from one second suturing hole 53 can extend into the next second suturing hole 53 along the second recessed groove 58, thereby avoiding the suture thread for suturing the blocking member 50 and the suture ring 10 from protruding from the blocking member 50 and being in contact with the first connecting arm 23, thereby avoiding affecting the movement of the first connecting arm 23.

In an embodiment, a plurality of first recessed grooves 34 and a plurality of first suturing holes 33 are arranged on a first circle C₁, and a plurality of second recessed grooves 58 and a plurality of second suturing holes 53 are arranged on a second circle C₂. The center of the first circle C₁, the center of the second circle C₂, and the center of the circle where the annular disk 30 is located are on the same straight line, and a radius of the first circle C₁ and a radius of the second circle C₂ are equal, so that the suture thread can smoothly extend through the junction of the annular disk 30 and the blocking member 50 via the first recessed grooves 34 and the second recessed grooves 58, thereby avoiding the suture thread being located outside the first recessed grooves 34 and the second recessed grooves 58 due to unequal radii of the first circle C₁ and the second circle C₂, thus avoiding affecting the mounting of the ventricular assist device.

The center of the first circle C₁, the center of the second circle C₂, and the center of the circle where the annular disk 30 is located are on the same straight line, and the radius of the first circle C₁ and the radius of the second circle C₂ are equal, so that there will be no abrupt change in radius at the junction of the first circle C₁ and the second circle C₂, making the suturing operation of the annular disk 30 and the blocking member 50 to the suture ring 10 smoother and simplifying the suturing operation. Moreover, the suture thread accommodated in the first recessed grooves 34 and the second recessed grooves 58 can form a circle from the top view of the ventricular connection mechanism 1, thereby improving the appearance consistency of the ventricular connection mechanism 1. The top view of the connection mechanism 1 refers to viewing the connection mechanism 1 along the central axis of the circle where the annular disk 30 is located. In this embodiment, widths of the first recessed groove 34 and the second recessed groove 58 are equal. The width of the first recessed groove 34 or the width of the second recessed groove 58 refers to a length of the first recessed groove 34 or a length of the second recessed groove 58 in the radial direction of the annular disk 30. In other embodiments, the widths of the first recessed groove 34 and the second recessed groove 58 may also be unequal.

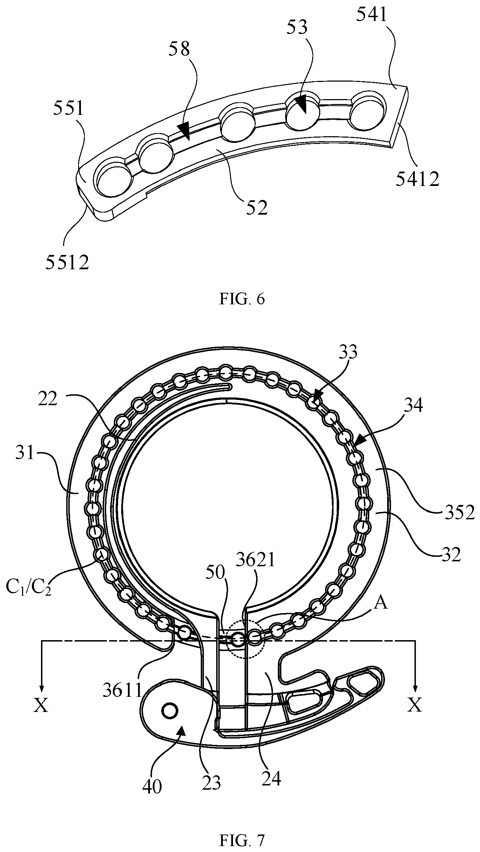

Referring to FIGS. 3 and 7, optionally, in an embodiment, the first end 361 has a first end surface 3611, and the second end 362 has a second end surface 3621. The first end surface 3611 and the second end surface 3621 are opposite to and spaced apart along the circumferential direction of the annular disk 30 to form the first opening 363.

At least one of the first end surface 3611 and the second end surface 3621 is tangent to an edge of the second suturing hole 53b located at the first opening 363.

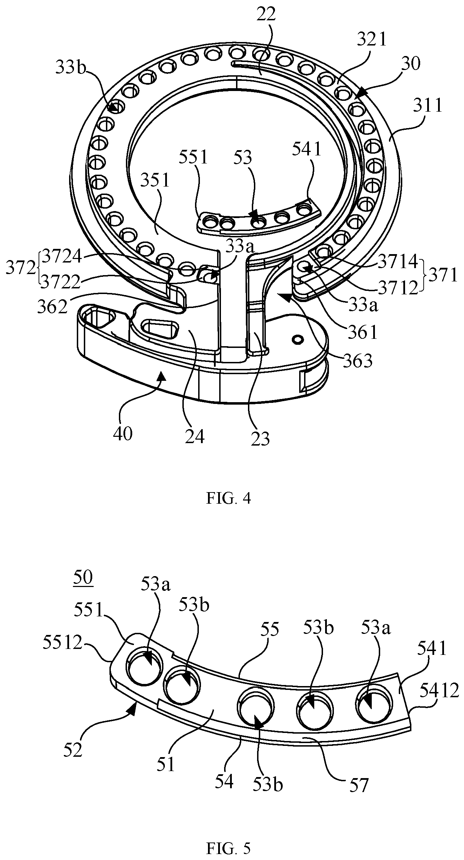

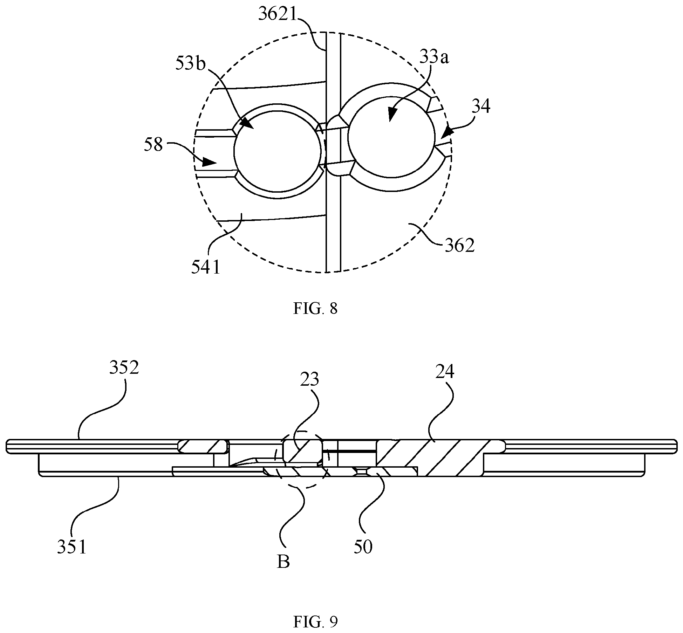

Referring to FIGS. 4, 7, and 8, in the illustrated embodiment, the second end surface 3621 is tangent to the edge of the second suturing hole 53b located at the first opening 363 and closer to the second end surface 3621. The second suturing hole 53b refers to the second suturing hole 53 closest to the second end surface 3621 among all second suturing holes 53 between the first end surface 3611 and the second end surface 3621. Thus, the suture thread extending through the first suturing hole 33a at the second end 362 can enter the second suturing hole 53b along the second end surface 3621, or the suture thread extending through the second suturing hole 53b can enter the first suturing hole 33a at the second end 362 along the second end surface 3621. On the one hand, the probability of the suture thread coming into contact with other structures at this location is reduced. On the other hand, the second end surface 3621 can support the suture thread, thereby reducing the probability of the suture thread being cut.

In one embodiment, the first end surface 3611 may also be tangent to the edge of the second suturing hole 53b located at the first opening 363 and closer to the first end surface 3611. Thus, the suture thread extending through the first suturing hole 33a can enter the corresponding second suturing hole 53b along the first end surface 3611, reducing the probability of the suture thread coming into contact with other structures at this location. Moreover, the first end surface 3611 can support the suture thread, thereby reducing the probability of the suture thread being cut, enabling the annular disk 30 and the blocking member 50 to be stably sutured to the suture ring 10. In another embodiment, both the first end surface 3611 and the second end surface 3621 may be tangent to the edge of the second suturing hole 53b located at the first opening 363.

Referring to FIGS. 1 to 5, in one embodiment, the annular disk 30 has a first surface 351 and a second surface 352 opposite to each other along the axial direction. The first surface 351 is located on the boss 321, and the first surface 351 is opposite to and connected to the second suturing surface 12 of the suture ring 10. The second surface 352 is located on the disk body 311. The blocking member 50 has a first connecting surface 51 and a second connecting surface 52 that are opposite to each other along the axial direction. The first connecting surface 51 is opposite to and connected to the second suturing surface 12 of the suture ring 10. The second connecting surface 52 is located on the side of the blocking member 50 away from the suture ring 10. Furthermore, the first connecting surface 51 is coplanar with the first surface 351, so that the junction between the blocking member 50 and the annular disk 30 can be sealed and attached to the suture ring 10 as much as possible, avoiding the gap between the junction between the blocking member 50 and the annular disk 30 and the suture ring 10, thereby further reducing the probability of blood leakage.

Optionally, in an embodiment, the first end 361 is provided with a first accommodating groove 371, and the second end 362 is provided with a second accommodating groove 372. The first accommodating groove 371 and the second accommodating groove 372 are both exposed on the first surface 351. The first accommodating groove 371 extends through the first end surface 3611, and the second accommodating groove 372 extends through the second end surface 3621.

The first connecting end 541 of the blocking member 50 is provided in the first accommodating groove 371, thereby limiting the movement of the first connecting end 541 relative to the annular disk 30 to a certain extent. The second connecting end 551 of the blocking member 50 is provided in the second accommodating groove 372, thereby limiting the movement of the second connecting end 551 relative to the annular disk 30 to a certain extent. Thus, while achieving the first connecting surface 51 of the blocking member 50 being coplanar with the first surface 351 of the annular disk 30, the connection strength between the blocking member 50 and the annular disk 30 can also be improved.

Furthermore, the blocking member 50 further has a first connecting side surface 54, a second connecting side surface 55, and a first connecting end surface 5412. The first connecting side surface 54 and the second connecting side surface 55 are two side surfaces of the blocking member 50 that are opposite in the radial direction of the annular disk 30. The first connecting end surface 5412 is provided at the first connecting end 541. The first connecting end surface 5412 is connected between the first connecting side surface 54 and the second connecting side surface 55. The first connecting side surface 54, the second connecting side surface 55, and the first connecting end surface 5412 are all connected to the first connecting surface 51.

The first connecting side surface 54 and the second connecting side surface 55 are both connected between the first connecting surface 51 and the second connecting surface 52. The connection between the first connecting surface 51 and the first connecting side 54 at the first connecting end 541 is provided with a rounded edge 57. By providing the rounded edge 57 at the first connecting end 541, sharp edges are avoided at the first connecting end 541, thereby preventing the operating personnel from being cut when suturing the blocking member 50 to the suture ring 10.

Furthermore, the first connecting end surface 5412 is in contact with a groove wall of the first accommodating groove 371, and the first connecting side surface 54 and the second connecting side surface 55 are exposed outside the first accommodating groove 371. That is, the first accommodating groove 371 has a first bottom wall 3712 and a first side wall 3714 connected to the first bottom wall 3712. The first bottom wall 3712 and the first side wall 3714 are both provided at the first end 361 of the annular disk 30. The first accommodating groove 371 is defined by the first bottom wall 3712 and the first side wall 3714. The first bottom wall 3712 is opposite to the first connecting surface 51, and the first side wall 3714 is opposite to and abuts against the first connecting end surface 5412. That is, the first accommodating groove 371 is a groove with an opening, and the side wall of the first accommodating groove 371 has no part opposite to the first connecting side surface 54 and the second connecting side surface 55, which can allow for appropriate dimensional errors of the blocking member 50. Therefore, when the second connecting end 551 is already provided in the second accommodating groove 372, the position of the first connecting end 541 relative to the first end 361 can be adjusted to enable the first connecting end 541 to be accommodated in the first accommodating groove 371, thereby the blocking member 50 can be fixedly connected to the annular disk 30. The situation where the first connecting end 541 cannot be accommodated in the first accommodating groove 371 due to deformation of the annular disk 30 or excessive width of the first connecting end 541 can be avoided.

The blocking member 50 further has a second connecting end surface 5512 provided at the second connecting end 551, and the second connecting end surface 5512 is connected to the first connecting surface 51. The first connecting side surface 54, the second connecting side surface 55, and the second connecting end surface 5512 are in contact with a groove wall of the second accommodating groove 372. That is, the second accommodating groove 372 has a second bottom wall 3722 and a second side wall 3724 connected to the second bottom wall 3722. The second accommodating groove 372 is defined by the second bottom wall 3722 and the second side wall 3724. The second bottom wall 3722 and the second side wall 3724 are both provided at the second end 362 of the annular disk 30. The second bottom wall 3722 is opposite to the second connecting surface 52, and the second side wall 3724 abuts against the first connecting side surface 54, the second connecting side surface 55, and the second connecting end surface 5512. Thus, the second connecting end 551 can be adapted to be accommodated in the second accommodating groove 372. That is, the shape of the second connecting end 551 is the same as that of the second accommodating groove 372, and the size of the second connecting end 551 is also the same as that of the second accommodating groove 372, so that the second connecting end 551 can just fill the second accommodating groove 372, so as to avoid the gap between the side wall of the second connecting end 551 and the groove wall of the second accommodating groove 372 after the second connecting end 551 is accommodated in the second accommodating groove 372, which can retain blood. In other words, thrombus caused by blood retention can be avoided.

Furthermore, the first connecting surface 51 at the second connecting end 551 is directly connected to the first connecting side surface 54, and the first connecting surface 51 is directly connected to the second connecting side surface 55. That is, the connection between the first connecting surface 51 and the first connecting side surface 54 at the second connecting end 551 is not rounded, and the connection between the first connecting surface 51 and the second connecting side surface 55 at the second connecting end 551 is also not rounded. Thus, the second connecting end 551 is seamlessly joined to the second end 362, which further facilitates sealed engagement between the junction of the blocking member 50 and the annular disk 30 and the suture ring 10, thereby further reducing the probability of blood leakage.

In other embodiments, the first end 361 may not be provided with the first accommodating groove 371, and the second end 362 may not be provided with the second accommodating groove 372. The first connecting end surface 5412 of the blocking member 50 can be directly and fixedly connected to the first end surface 3611, and the second connecting end surface 5512 can be directly and fixedly connected to the second end surface 3621, which can also achieve fixed connection between the blocking member 50 and the annular disk 30.

Referring to FIGS. 2 and 3, optionally, in an embodiment, the side of the first connecting arm 23 adjacent to the blocking member 50 is provided with an avoidance groove 231, so that the first connecting arm 23 is spaced apart from the blocking member 50. For example, a gap is formed between the blocking member 50 and the bottom surface of the avoidance groove 231. The avoidance groove 231 extends through the opposite sides of the first connecting arm 23 along the circumferential direction of the annular disk 30 to better avoid the blocking member 50. By providing the avoidance groove 231 on the side of the first connecting arm 23 adjacent to the blocking member 50, the first connecting arm 23 can avoid the blocking member 50, reducing the probability of the first connecting arm 23 coming into contact with the blocking member 50 during movement and improving the smoothness of the operation of the ventricular connection mechanism 1.

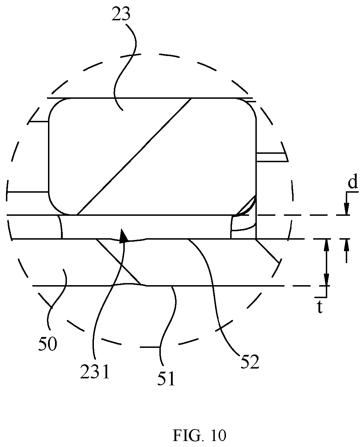

Further, referring to FIGS. 3, 9, and 10, the second connecting surface 52 of the blocking member 50 is opposite to and parallel to the bottom surface of the avoidance groove 231. A distance d between the second connecting surface 52 and the bottom surface of the avoidance groove 231 and a thickness t of the blocking member 50 satisfies: 0.3≤d/t≤0.7. The thickness t of the blocking member 50 refers to the maximum distance between the first connecting surface 51 and the second connecting surface 52, that is, the thickness t is the maximum thickness of the blocking member 50. In this way, while ensuring the strength of the blocking member 50, there is a sufficient gap between the blocking member 50 and the bottom surface of the avoidance groove 231, so as to prevent the first connecting arm 23 from being in contact with the blocking member 50. When d/t is less than 0.3, with the thickness t of the blocking member 50 remaining unchanged, the distance d between the second connecting surface 52 and the bottom surface of the avoidance groove 231 is relatively small, and the first connecting arm 23 may come into contact with the blocking member 50 during the movement. When d/t is greater than 0.7, with the thickness t of the blocking member 50 remaining unchanged, the distance d between the second connecting surface 52 and the bottom surface of the avoidance groove 231 is relatively large, making the second connecting arm 24 relatively thin and increasing the probability of breakage of the second connecting arm 24, or, with the distance d between the second connecting surface 52 and the bottom surface of the avoidance groove 231 remaining unchanged, the blocking member 50 is relatively thin, reducing the bending strength of the blocking member 50.

The present application also provides a ventricular assist system, which includes a ventricular assist device (not shown) and the ventricular connection mechanism 1 of any of the above embodiments. The ventricular assist device includes an inlet pipe capable of extending through the clamping ring 22, and the clamping ring 22 can clamp the inlet pipe. The locking assembly 40 is connected to both the first connecting arm 22 and the second connecting arm 23. Thus, by driving the clamping ring 22 to contract through the locking assembly 40, the clamping ring 22 can be driven to clamp the inlet pipe along the circumferential direction of the annular disk 30, thereby fixing the ventricular assist device onto the heart tissue.

The above embodiments are only used to illustrate the technical solutions of the present application and are not intended to limit the present application. Although the present application has been described in detail with reference to the foregoing embodiments, those skilled in the art should understand that modifications can still be made to the technical solutions described in the foregoing embodiments, or equivalent substitutions can be made to some of the technical features. Such modifications or substitutions do not cause the essence of the corresponding technical solutions to deviate from the spirit and scope of the technical solutions of the embodiments of the present application, and should all fall within the protection scope of the present application.

## Claims

1. A ventricular connection mechanism, comprising:
a suture ring;
a clamping assembly comprising an annular disk and a clamping ring fixedly connected to the annular disk, the annular disk having a first opening, the clamping ring having a second opening, a size of the second opening being adjustable, and an inner diameter of the clamping ring being adjustable by adjusting the size of the second opening; and
a blocking member connected to the annular disk, a position of the blocking member corresponding to a position of the first opening, such that the blocking member and the annular disk cooperatively form a closed annular structure, the annular structure being provided with a fixing structure capable of fixedly connecting the annular structure to the suture ring, and the fixing structure being provided around an inner hole of the suture ring.

2. The ventricular connection mechanism according to claim 1, wherein the blocking member is arc-shaped, and a central axis of a circle corresponding to an arc where the blocking member is located is coaxial with a central axis of a circle where the annular disk is located.

3. The ventricular connection mechanism according to claim 1, wherein the annular disk is provided with a plurality of first suturing holes, the blocking member is provided with a plurality of second suturing holes, and the ventricular connection mechanism further comprises a suture thread extending through the plurality of first suturing holes, the plurality of second suturing holes, and the suture ring, so as to fix the annular structure to the suture ring, wherein the plurality of first suturing holes, the plurality of second suturing holes, and the suture thread cooperatively constitute the fixing structure.

4. The ventricular connection mechanism according to claim 3, wherein the annular disk is further provided with a plurality of first recessed grooves capable of accommodating the suture thread, each first recessed groove is provided between adjacent two first suturing holes, and the first recessed groove is in communication with adjacent two first suturing holes; and/or the blocking member is further provided with a plurality of second recessed grooves capable of accommodating the suture thread, each second recessed groove is provided between adjacent two second suturing holes, and the second recessed groove is in communication with adjacent two second suturing holes.

5. The ventricular connection mechanism according to claim 4, wherein the plurality of first recessed grooves and the plurality of first suturing holes are arranged on a first circle, the plurality of second recessed grooves and the plurality of second suturing holes are arranged on a second circle, a center of the first circle, a center of the second circle, and a center of a circle where the annular disk is located are on a same straight line, and a radius of the first circle and a radius of the second circle are equal.

6. The ventricular connection mechanism according to claim 3, wherein the annular disk further has a first end surface and a second end surface, the first end surface and the second end surface are opposite to and spaced apart, so as to form the first opening;
wherein the first end surface is tangent to an edge of the second suturing hole located at the first opening and closer to the first end surface; and/or
wherein the second end surface is tangent to an edge of the second suturing hole located at the first opening and closer to the second end surface.

7. The ventricular connection mechanism according to claim 3, wherein the annular disk further has a first end and a second end, the first end and the second end are spaced apart to form the first opening, each of the first end and the second end is provided with the first suturing hole, the blocking member comprises a first connecting end and a second connecting end opposite to each other, each of the first connecting end and the second connecting end is provided with the second suturing hole;
wherein the first suturing hole at the first end is coaxial with the second suturing hole at the first connecting end; and/or;
wherein the first suturing hole at the second end is coaxial with the second suturing hole at the second connecting end.

8. The ventricular connection mechanism according to claim 1, wherein the annular disk has a first surface opposite to the suture ring, the blocking member has a first connecting surface opposite to the suture ring, and the first connecting surface is coplanar with the first surface.

9. The ventricular connection mechanism according to claim 8, wherein the annular disk further has a first end and a second end, the first end and the second end are spaced apart to form the first opening, the first end is provided with a first accommodating groove, the second end is provided with a second accommodating groove, both the first accommodating groove and the second accommodating groove are exposed on the first surface, the blocking member comprises a first connecting end and a second connecting end opposite to each other, the first connecting end is provided in the first accommodating groove, and the second connecting end is provided in the second accommodating groove.

10. The ventricular connection mechanism according to claim 9, wherein the blocking member further has a first connecting end surface provided at the first connecting end, the first connecting end surface is connected to the first connecting surface the first accommodating groove has a first bottom wall and a first side wall connected to the first bottom wall, the first accommodating groove is defined by the first bottom wall and the first side wall, the first bottom wall is opposite to the first connecting surface, and the first side wall is opposite to and abuts against the first connecting end surface; and/or
wherein the blocking member further has a first connecting side surface, a second connecting side surface, and a second connecting end surface provided at the second connecting end, the first connecting side surface, the second connecting side surface, and the second connecting end surface are connected to the first connecting surface, the first connecting side is opposite to the second connecting side, the second accommodating groove has a second bottom wall and a second side wall connected to the second bottom wall, the second accommodating groove is defined by the second bottom wall and the second side wall, the second bottom wall is opposite to the first connecting surface, and the second side wall abuts against the first connecting side surface, the second connecting side surface, and the second connecting end surface.

11. The ventricular connection mechanism according to claim 1, wherein the clamping assembly further comprises a first connecting arm and a second connecting arm, the clamping ring is provided on an inner periphery of the annular disk the first connecting arm is connected to one end of the clamping ring and extends outward from the first opening, the second connecting arm is connected to another end of the clamping ring and is connected to the annular disk, and the first connecting arm is capable of approaching or moving away from the second connecting arm to adjust the size of the second opening.

12. The ventricular connection mechanism according to claim 11, wherein the blocking member is located between the suture ring and the first connecting arm, and a side of the first connecting arm adjacent to the blocking member is provided with an avoidance groove, so as to space the first connecting arm from the blocking member.

13. The ventricular connection mechanism according to claim 12, wherein the blocking member has a first connecting surface and a second connecting surface that are opposite to each other, the first connecting surface is opposite to the suture ring the second connecting surface is opposite to and parallel to a bottom surface of the avoidance groove and a distance d between the second connecting surface and the bottom surface of the avoidance groove and a thickness t of the blocking member satisfy: 0.3≤d/t≤0.7.

14. The ventricular connection mechanism according to claim 1, wherein the clamping assembly further comprises a first connecting arm and a second connecting arm, the first connecting arm and the second connecting arm are connected to two ends of the clamping ring in a circumferential direction of the clamping ring, respectively, the ventricular connection mechanism further comprises a locking assembly, the locking assembly comprises a base body and a locking member, the base body is connected to the second connecting arm, one end of the locking member is rotatably connected to the base body, another end of the locking member is provided with an engaging portion configured to be engaged with the second connecting arm, when the locking member rotates relative to the base body, the first connecting arm is pushed to move toward the second connecting arm, so as to reduce the second opening and reduce the inner diameter of the clamping ring.

15. A ventricular assist system, comprising a ventricular assist device and the ventricular connection mechanism according to any one of claims 1 to 14, the ventricular assist device having an inlet pipe capable of extending through the clamping ring, and the clamping ring being capable of clamping the inlet pipe.
